# EUROPEAN PATENT APPLICATION

(11) **EP 4 190 362 A1**
(43) Date of publication of application: **07.06.2023**
(21) Application number: 21850664.0
(22) Date of filing: 29.07.2021
(51) Int. Cl.: A61K 48/00, A61K 35/761, A61K 39/395, A61K 45/00, A61P 35/00, A61P 43/00, C12N 15/12, C12N 15/861

(54) **AGENT INCLUDING REIC/DKK-3 GENE FOR TREATING EGFR GENE MUTATION POSITIVE LUNG CANCER**

(30) Priority: 31.07.2020 JP 2020130701
(71) Applicant: Momotaro-Gene Inc., Okayama-shi, Okayama 700-8558 (JP)
(72) Inventor: KUMON Hiromi, Okayama-shi, Okayama 700-0904 (JP); OHASHI Kadoaki, Okayama-shi, Okayama 700-8530 (JP); KIURA Katsuyuki, Okayama-shi, Okayama 700-8530 (JP); MAEDA Yoshinobu, Okayama-shi, Okayama 700-8530 (JP); NAKASUKA Takamasa, Okayama-shi, Okayama 700-8530 (JP); NISHII Kazuya, Okayama-shi, Okayama 700-8530 (JP)
(74) Representative: Grünecker Patent- und Rechtsanwälte PartG mbB
(86) International application number: PCT/JP2021/028091
(87) International publication number: WO 2022/025178

(57) **Abstract**

This invention provides a therapeutic method for treatment of EGFR gene mutation-positive lung cancer using the REIC/Dkk-3 gene in combination with the EGFR tyrosine kinase inhibitor, the PD-1/PD-L1 immune checkpoint pathway inhibitor, and/or the molecular-targeted agent. This invention provides a therapeutic agent comprising, as an active ingredient, the REIC/Dkk-3 gene, which is used in combination with the EGFR tyrosine kinase inhibitor, the PD-1/PD-L1 immune checkpoint pathway inhibitor, and/or the molecular-targeted agent for treatment of EGFR gene mutation-positive lung cancer.

## Description

### Technical Field

The present invention relates to a combinatorial method for treatment of EGFR gene mutation-positive lung cancer using the REIC/Dkk-3 gene in combination with the EGFR tyrosine kinase inhibitor. The present invention also relates to a combinatorial method for treatment of EGFR gene mutation-positive lung cancer using an immune checkpoint inhibitor in combination with a molecular-targeted agent.

### Background Art

The REIC/Dkk-3 gene has been known to be involved in cell immortalization. It has been reported that expression of the REIC/Dkk-3 gene is inhibited in cancer cells. In addition, it has been reported that the REIC/Dkk-3 gene is used in cancer treatment (Patent Document 1).

Immune checkpoint inhibitors (ICIs), such as the anti-PD-1 (programmed cell death 1) antibody and the anti-PD-L1 (programmed cell death ligand 1) antibody, which are the PD-1/PD-L1 immune checkpoint pathway inhibitors, are known to be effective against various malignant tumors.

The EGFR tyrosine kinase inhibitor (EGFR-TKI) is the first-line agent for the drug treatment of the epidermal growth factor receptor (EGFR) gene mutation-positive lung cancer; however, EGFR gene mutation-positive lung cancer would not be cured, and drug tolerance would be eventually developed. Accordingly, development of a novel therapeutic strategy is necessary. In recent years, immune checkpoint inhibitors (ICIs) that would exert innovative effects have been used in clinical settings, and advanced lung cancer is expected to be cured. Unfortunately, effects of ICI on EGFR gene mutation-positive lung cancer, which is often observed in non-smokers, are poor (Non-Patent Documents 1 and 2), and use of cytotoxic anti-tumor agents is the primary treatment after patients acquire tolerance. While subset analyses of prospective clinical trials indicate a possibility of ICI for exerting effects on EGFR gene mutation-positive lung cancer in combination with cytotoxic anti-tumor agents and anti-VEGF antibodies, at present, a role of ICI on EGFR gene mutation-positive lung cancer has not yet been verified.

While EGFR gene mutation-positive lung cancer is effectively treated with EGFR-TKI, EGFR gene mutation-positive lung cancer would not be cured, and treatment tolerance would be developed in the end. Examples of currently available treatment options after the development of EGFR-TKI-tolerance include cytotoxic anti-tumor agents and ICI, such as the anti-PD-1 antibody. While effective use of ICI may lead to long-term survival, effects of ICI on EGFR gene mutation-positive lung cancer are known to be poor (Non-Patent Document 1). EGFR gene mutation-positive lung cancer is reported to have a low tumor mutation burden (Non-Patent Document 3) and a small number of CD8+ tumor-infiltrating lymphocytes (Non-Patent Document 4). Accordingly, low immunogenicity is deduced to be a cause of poor effects of ICI. Therefore, a critical objective of lung cancer treatment is to enhance immunogenicity to improve the effects of ICI.

Development of tumor immunotherapy of EGFR gene mutation-positive lung cancer is in urgent need. While mouse models carrying pathological conditions of human EGFR gene mutation-positive lung cancer are important for evaluation of the immune system against tumors that dynamically changes, the number of such models is not sufficient, and tumor immunity against EGFR gene mutation-positive lung cancer has not yet been fully examined.

The group of Harvard University and Memorial Sloan Kettering Cancer Center (U.S.A.) prepared lung cancer mouse models into which the EGFR gene mutation had been introduced using a CCSP promoter targeting mouse lung Clara cells. The group reported that PD-L1 expression is induced by the EGFR signal in mouse lung cancer and the anti-PD-1 antibody is effectively used in treatment thereof (Non-Patent Document 5). However, a plurality of clinical trials demonstrate that, in clinical practice and in the case of human patients, the PD-L1 expression level in lung cancer with the EGFR gene mutation is low and effects of the anti-PD-1 antibody are poor (Non-Patent Documents 1 and 2).

Under the above circumstances, the lung cancer mouse models of the group may not adequately reproduce the actual lung adenocarcinoma with the EGFR gene mutation in human patients. In order to perform preclinical studies on tumor immunity against the lung cancer with the EGFR gene mutation, accordingly, animal models carrying properties that are very similar to the findings obtained in human samples are very important.

In order to understand the pathological conditions of lung cancer with the EGFR gene mutation, the present inventors established the immunocompetent C57BL/6-based Egfr (mouse EGFR) gene-modified (Egfr gene mutation positive) lung cancer mouse (C57BL/6/EgfrdelE748-A752) with the use of the alveolar epithelial type II cell-specific SPC promoter, and a patent was granted therefor (Non-Patent Document 6 and Patent Document 2). Also, they removed a tumor from the Egfr gene mutation-positive lung cancer-carrying mouse and verified that the tumor would survive subcutaneously in other C57BL/6J mice and the tumor would be repeatedly subcultured (Non-Patent Document 7). In the EGFR gene mutation-positive lung cancer-carrying mouse, the alveolar epithelial type II cell-specific Egfr gene mutation is expressed to develop the Egfr gene mutation-dependent lung tumor. While EGFR-TKI is effectively used as with the case of human lung cancer, the tumor would not be cured, and drug tolerance would be developed. In the case of the mouse lung tumor indicated above, PD-L1 is not expressed, and effects of the anti-PD-1 antibody and the anti-PD-L1 antibody are poor. Such features are consistent with those of human EGFR gene mutation-positive lung cancer. That is, such animal models are considered to accurately carry the pathological conditions of interest.

Meanwhile, the antibody against the vascular endothelial growth factor receptor (VEGFR) is reported to significantly function to normalize blood vessels at a low dose to thereby promoting effector T cell infiltration (Non-Patent Document 8).

### Prior Art Documents

### Patent Documents

Patent Document 1: WO 01/038528
Patent Document 2: JP Patent No. 5,255,216

### Non-Patent Documents

Non-Patent Document 1: Lee et al., J. Thorac. Oncol., February 01, 2017, vol. 12, no. 2, 403-407
Non-Patent Document 2: Garon et al., N. Engl. J. Med., May 21, 2015, vol. 372, no. 21, 2018-2028
Non-Patent Document 3: Offin et al., Clin. Cancer Res., February 01, 2019, vol. 25, no. 3, 1063-1069
Non-Patent Document 4: Sugiyama et al., Sci. Immunol., January 31, 2020, vol. 5, no. 43, eaav3937.
Non-Patent Document 5: Akbay et al., Cancer Discov., December, 2013, vol. 3, no. 12, 1355-1363
Non-Patent Document 6: Ohashi et al., Cancer Sci., September, 2008, vol. 99, no. 9, 1747-1753
Non-Patent Document 7: Higo et al., Lung Cancer, October 01, 2019, vol. 136, 86-93
Non-Patent Document 8: Huanga et al., PNAS, October 23, 2012, vol. 109, no. 43, 17561-17566

### Summary of the Invention

### Objects to Be Attained by the Invention

The present invention provides a method for treatment of EGFR gene mutation-positive lung cancer with the use of the REIC/Dkk-3 gene in combination with the EGFR tyrosine kinase inhibitor, the immune checkpoint inhibitor, the molecular-targeted agent. Means for Attaining the Objects

The present inventors examined the effects of use of REIC/Dkk-3 in combination with the EGFR tyrosine kinase inhibitor (EGFR-TKI), use of REIC/Dkk-3 in combination with the immune checkpoint inhibitor, and use of REIC/Dkk-3 in combination with the immune checkpoint inhibitor and the molecular-targeted agent on EGFR gene mutation-positive lung cancer.

The present inventors discovered that EGFR gene mutation-positive lung cancer could be treated with the use of REIC/Dkk-3 in combination with the EGFR tyrosine kinase inhibitor and with the further use of the immune checkpoint inhibitor and the molecular-targeted agent in combination. They also discovered that use of such agents in combination would provide a novel method of tumor immunotherapy for EGFR gene mutation-positive lung cancer on which effects of the immune checkpoint inhibitor, such as the anti-PD-1 antibody, which is the PD-1/PD-L1 immune checkpoint pathway inhibitor, would be poor. This indicates that use of REIC/Dkk-3 in combination with other therapeutic agents is effective on treatment of EGFR gene mutation-positive lung cancer.

Specifically, the present invention is as described below.
[1] A therapeutic agent comprising, as an active ingredient, the REIC/Dkk-3 gene, which is used in combination with the EGFR tyrosine kinase inhibitor for treatment of EGFR gene mutation-positive lung cancer.
[2] A therapeutic agent comprising, as an active ingredient, the REIC/Dkk-3 gene, which is used in combination with the immune checkpoint inhibitor and/or the molecular-targeted agent for treatment of EGFR gene mutation-positive lung cancer.
[3] The therapeutic agent according to [1] or [2], wherein the target EGFR gene mutation-positive lung cancer is a primary cancer.
[4] A therapeutic agent comprising, as an active ingredient, the REIC/Dkk-3 gene, which is used in combination with the immune checkpoint inhibitor for treatment of EGFR gene mutation-positive lung cancer and is to be administered to a patient of the primary EGFR gene mutation-positive lung cancer after the combinatorial treatment using the REIC/Dkk-3 gene in combination with the EGFR tyrosine kinase inhibitor.
[5] A therapeutic agent comprising, as an active ingredient, the REIC/Dkk-3 gene, which is used in combination with the immune checkpoint inhibitor and the molecular-targeted agent for treatment of EGFR gene mutation-positive lung cancer and is to be administered to a patient of the primary EGFR gene mutation-positive lung cancer after the combinatorial treatment using the REIC/Dkk-3 gene in combination with the EGFR tyrosine kinase inhibitor.
[6] A therapeutic agent comprising, as an active ingredient, the REIC/Dkk-3 gene, which is used in combination with the immune checkpoint inhibitor for treatment of recurrent EGFR gene mutation-positive lung cancer after the treatment using the EGFR tyrosine kinase inhibitor.
[7] A therapeutic agent comprising, as an active ingredient, the REIC/Dkk-3 gene, which is used in combination with the immune checkpoint inhibitor and the molecular-targeted agent for treatment of recurrent EGFR gene mutation-positive lung cancer after the treatment using the EGFR tyrosine kinase inhibitor.
[8] The therapeutic agent according to any of [2], [3], [5], and [7], wherein the molecular-targeted agent is the anti-VEGFR antibody.
[9] The therapeutic agent according to any of [2] to [8], wherein the immune checkpoint inhibitor is the PD-1/PD-L1 immune checkpoint pathway inhibitor.
[10] The therapeutic agent according to any of [1] to [9], wherein the REIC/Dkk-3 gene is integrated into an adenovirus vector.
[11] The therapeutic agent according to any of [1] to [10], which is in the form of a local administration agent.
[12] A kit of combinatorial therapeutic agents comprising the REIC/Dkk-3 gene in combination with the EGFR tyrosine kinase inhibitor, which is used for treatment of EGFR gene mutation-positive lung cancer.
[13] A kit of combinatorial therapeutic agents comprising the REIC/Dkk-3 gene, the immune checkpoint inhibitor, and/or the molecular-targeted agent in combination, which is used for treatment of EGFR gene mutation-positive lung cancer.
[14] The kit of combinatorial therapeutic agents according to [12] or [13], wherein the target EGFR gene mutation-positive lung cancer is a primary cancer.
[15] A kit of combinatorial therapeutic agents comprising the REIC/Dkk-3 gene in combination with the immune checkpoint inhibitor, which is to be administered to a patient of the primary EGFR gene mutation-positive lung cancer after the combinatorial treatment using the REIC/Dkk-3 gene in combination with the EGFR tyrosine kinase inhibitor.
[16] A kit of combinatorial therapeutic agents comprising the REIC/Dkk-3 gene, the immune checkpoint inhibitor, and the molecular-targeted agent in combination, which is to be administered to a patient of the primary EGFR gene mutation-positive lung cancer after the combinatorial treatment using the REIC/Dkk-3 gene in combination with the EGFR tyrosine kinase inhibitor.
[17] A kit of combinatorial therapeutic agents comprising the REIC/Dkk-3 gene in combination with the immune checkpoint inhibitor, which is used for treatment of recurrent EGFR gene mutation-positive lung cancer after the treatment using the EGFR tyrosine kinase inhibitor.
[18] A kit of combinatorial therapeutic agents comprising the REIC/Dkk-3 gene, the immune checkpoint inhibitor, and the molecular-targeted agent in combination, which is used for treatment of recurrent EGFR gene mutation-positive lung cancer after the treatment using the EGFR tyrosine kinase inhibitor.
[19] The kit of combinatorial therapeutic agents according to any of [13], [14], [16], and [18], wherein the molecular-targeted agent is the anti-VEGFR antibody.
[20] The kit of combinatorial therapeutic agents according to any of [13] to [19], wherein the immune checkpoint inhibitor is the PD-1/PD-L1 immune checkpoint pathway inhibitor.
[21] The kit of combinatorial therapeutic agents according to any of [12] to [20], wherein the REIC/Dkk-3 gene is integrated into an adenovirus vector.
[22] The kit of combinatorial therapeutic agents according to any of [12] to [21], which is in the form of a local administration agent.

This description includes part or all of the content as disclosed in the description and/or drawings of Japanese Patent Application No. 2020-130701, which is a priority document of the present application.

### Effects of the Invention

While EGFR gene mutation-positive lung cancer is effectively treated with EGFR-TKI, EGFR gene mutation-positive lung cancer would not be cured, and treatment tolerance would be developed in the end. Effects of immune checkpoint inhibitors, such as the anti-PD-1 antibody, on EGFR gene mutation-positive lung cancer are poor. REIC/Dkk-3 has effects of inducing cancer-cell-specific cell death and effects of immune activation. Such effects are different from the effects attained with the use of conventional cytotoxic anti-tumor agents and molecular-targeted agents. With the use of REIC/Dkk-3 in combination with the EGFR tyrosine kinase inhibitor and the further use of the PD-1/PD-L1 immune checkpoint pathway inhibitor and the molecular-targeted agent in combination, the immunity against tumors can be activated, and local anti-tumor effects can be potentiated. By the method of treatment using therapeutic agents in combination, the immunity against not only a localized tumor but also tumors throughout the body can be activated, and the anti-tumor effects can be potentiated.

### Brief Description of the Drawings

Fig. 1 shows methods of administration of therapeutic agents in Experiments (Experiment 1, Experiment 2, Experiment 3, and Experiment 4) performed in Examples.
Fig. 2 shows the effects attained when Gefitinib is administered in combination with an adenovirus vector comprising the REIC/Dkk-3 gene to the tumor mouse model of Experiment 1.
Fig. 3 shows the effects attained when an adenovirus vector comprising the REIC/Dkk-3 gene is administered to the tumor mouse model of Experiment 2. Fig. 3A shows changes in tumor volumes on the administration side and Fig. 3B show changes in tumor volumes on the other side.
Fig. 4 shows the effects attained when an adenovirus vector comprising the REIC/Dkk-3 gene is administered in combination with the anti-PD-1 antibody to the tumor mouse model of Experiment 3. Fig. 4A shows changes in tumor volumes on the administration side and Fig. 4B show changes in tumor volumes on the other side.
Fig. 5 shows the effects attained when an adenovirus vector comprising the REIC/Dkk-3 gene, the anti-PD-1 antibody, and the anti-VEGFR antibody are administered in combination to the tumor mouse model of Experiment 4. Fig. 5A shows changes in tumor volumes on the administration side and Fig. 5B show changes in tumor volumes on the other side.
Fig. 6 shows an example of a structure of Ad-REIC/Dkk-3.
Fig. 7 shows a sequence of Ad-REIC/Dkk-3.

### Embodiments of the Invention

Hereafter, the present invention is described in detail.

In the method of treatment using therapeutic agents in combination according to the present invention, the REIC/Dkk-3 gene is used in combination with the EGFR tyrosine kinase inhibitor, and the PD-1/PD-L1 immune checkpoint pathway inhibitor and the molecular-targeted agent are further used in combination.

The nucleotide sequence of REIC/Dkk-3 DNA is shown in SEQ ID NO: 1 of Sequence Listing. The amino acid sequence of the REIC/Dkk-3 protein encoded by REIC/Dkk-3 DNA is shown in SEQ ID NO: 2 of Sequence Listing. DNA having at least 85%, preferably at least 90%, more preferably at least 95%, and particularly preferably at least 97% sequence identity to the nucleotide sequence represented by SEQ ID NO: 1 when calculated with the use of, for example, BLAST (Basic Local Alignment Search Tool at the National Center for Biological Information (NCBI)) (using, for example, default parameters) is within the scope of the REIC/Dkk-3 DNA.

A fragment nucleotide of REIC/Dkk-3 can also be used. Examples of nucleotide sequences comprising nucleotide 1 to any of nucleotides 117 to 234 of the nucleotide sequence of REIC/Dkk-3 DNA shown in SEQ ID NO: 1 include a polynucleotide comprising nucleotides 1 to 117 (SEQ ID NO: 3) and a polynucleotide comprising nucleotides 1 to 234 (SEQ ID NO: 4).

The REIC/Dkk-3 gene can be introduced into a subject in accordance with a conventional technique. Examples of methods of gene transfection into a subject include a method involving the use of a virus vector and a method involving the use of a non-virus vector.

The REIC/Dkk-3 gene can be introduced into a cell or tissue without the use of the virus described above. The REIC/Dkk-3 gene can be introduced with the use of a recombinant expression vector comprising a gene expression vector introduced thereinto, such as a plasmid vector.

Examples of typical virus vectors used for gene transfection include adenovirus, adeno-associated virus, and retrovirus vectors. The target gene may be introduced into a DNA or RNA virus, such as a detoxicated retrovirus, herpesvirus, vaccinia virus, pox virus, polio virus, Sindbis virus, hemagglutinating virus of Japan (HVJ), SV40, or immunodeficiency virus (HIV), to infect cells with such recombinant virus. Thus, the target gene can be introduced into cells. Use of an adenovirus vector is preferable.

A vector comprises a construct containing the REIC/Dkk-3 gene. A construct containing the REIC/Dkk-3 DNA may adequately comprise a promoter, an enhancer for gene transcription, a poly A signal, a marker gene used for labeling and/or selection of cells transfected with a gene, or the like. In such a case, a conventional promoter can be used.

Such a construct comprises the REIC/Dkk-3 gene, at least 1 first promoter, a poly A addition sequence downstream of the first promoter, and an enhance or a second promoter ligated to a site downstream of the DNA construct.

A promoter comprises a particular nucleotide sequence in DNA that initiates transcription using DNA as a template. In general, promoters comprise common sequences. For example, a prokaryotic organism, such as *E. coli,* generally comprises the sequence TATAATG located at the -10bp position from the transcription initiation site and the sequence TTGACA located at the -35bp position therefrom. An eukaryotic organism generally comprises the TATA box located at the -20bp position therefrom. It is necessary that the expression cassette of the present invention comprise a first promoter upstream of the target gene. The expression cassette of the present invention may comprise a second promoter downstream of the target gene. Promoters used as the first promoter and the second promoter are not particularly limited, and the first promoter may be the same with or different from the second promoter. Specific or selective promoters, such as non-specific promoter that can promote foreign gene expression in any cell or tissue, a tissue- or organ-specific promoter, a tumor-specific promoter, or a development- or differentiation-specific promoter, can be used. For example, a specific promoter can be used as a first promoter, and a non-specific promoter can be used as a second promoter. Examples of promoters that can be used in the present invention include: cancer- or tumor-specific promoters, such as human telomerase reverse transcriptase (hTERT), prostate-specific antigen (PSA), c-myc, and GLUT promoters; ES cell and cancer stem cell-specific promoters, such as OCT3/4 and NANOG promoters; neural stem cell-specific promoters, such as Nestin promoter; cell stress-sensitive promoters, such as HSP70, HSP90, and p53 promoters; hepatic cell-specific promoters, such as albumin promoter; radiation-sensitive promoters, such as TNF-alpha promoter; promoters increasing the infected plasmid copy number, such as SV40 promoter; and proliferative cell-specific promoters, such as EF1-alpha promoter. More specific examples of first promoters that can be used include CMV-i promoter (hCMV + intron promoter), β actin promoter, CAG promoter, and CMV promoter, and a more specific example of a second promoter that can be used is CMV promoter. An origin animal species of a β actin promoter is not particularly limited, and mammalian β actin promoters, such as a human β actin promoter or a chicken actin promoter, can be used. An artificial hybrid promoter, such as the aforementioned CMV-i promoter, can also be used. The CMV-i promoter can be synthesized on the basis of, for example, U.S. Patent No. 5,168,062 or 5,385,839. A promoter may be a part of a core promoter consisting of a minimal sequence having promoter activity. A core promoter is a promoter region having activity of inducing accurate transcription initiation and it may comprise the TATA box. Among the promoters described above, a cancer- and/or tumor-specific promoter, such as hTERT promoter, targets cancer. Such promoter can be thus preferably used for gene therapy or cancer diagnosis based on gene expression.

An origin of a poly A addition sequence (polyadenylation sequence, poly A) is not limited, and examples include poly A addition sequences derived from a growth hormone gene, such as a poly A addition sequence derived from a bovine growth hormone gene, a poly A addition sequence derived from a human growth hormone gene, a poly A addition sequence derived from SV40 virus, and a poly A addition sequence derived from a human or rabbit β globulin gene. By integrating a poly A addition sequence into an expression cassette, transcription efficiency is increased. The nucleotide sequence of the BGA poly A addition sequence is shown in a sequence after nucleotide 13 of the nucleotide sequence represented by SEQ ID NO: 5.

An enhancer is not limited, provided that it can increase the amount of messenger RNA (mRNA) generated by transcription. An enhancer comprises a nucleotide sequence having effects of accelerating promoter activity. In general, an enhancer is of approximately 100 bp. An enhancer can accelerate transcription regardless of the sequence direction. In the present invention, a single type of an enhancer may be used, a plurality of enhancers of the same type may be used, or a plurality of enhancers of different types may be used in combination. When a plurality of enhancers of different types are used, the order of the use is not limited. For example, CMV enhancer, SV40 enhancer, or telomerase reverse transcriptase (hTERT) enhancer can be used. For example, an enhancer may be composed of a sequence comprising hTERT enhancer, SV40 enhancer, and CMV enhancer ligated in that order.

A plurality of enhancers, such as 1 to 4 enhancers, may be ligated to a site upstream of a DNA construct comprising DNA encoding a target protein and a poly A addition sequence. While an enhancer to be ligated to an upstream site is not limited, CMV enhancer is preferable. An example is a 4 × CMV enhancer comprising 4 CMV enhancers ligated to each other.

When an enhancer is inserted into a site immediately downstream of the DNA construct comprising "promoter-target gene-poly A addition sequence," a protein of the target gene can be expressed at a higher level compared with a conventional gene expression system.

With the use of the CMV i promoter in combination with the CMV enhancer, in particular, a protein of the target gene can be expressed at a high level upon insertion of any gene into substantially any of the cells (host cells) with the use of any transfection reagent.

RU5' may be ligated to a site immediately upstream of DNA encoding a target protein. When RU5' is ligated to a site "immediately upstream," RU5' is directly ligated without the aid of an element having other particular functions. A short sequence may be inserted therebetween as a linker. RU5' is HTLV-derived LTR, which is an element that increases the protein expression level upon insertion thereof (Mol. Cell. Biol., Vol. 8 (1), pp. 466-472, 1988). When RU5' is inserted into a site opposite from the site as reported, effects of a promoter for increasing an expression level by enhancer introduction may be cancelled.

In addition, UAS may be ligated to a site immediately upstream of the enhancer and/or the promoter. UAS is a region to which the GAL4 gene binds. By inserting the GAL4 gene later, a protein expression level is increased.

Further, SV40-ori may be ligated to the uppermost region of the expression cassette. SV40-ori is a region to which the SV40 gene binds. By inserting the SV40 gene, a protein expression level is increased. It is necessary that the elements described above be operably linked to each other. When such elements are operably linked to each other, each of such elements exerts its functions to increase the expression level of the target gene.

The DNA construct comprises REIC/Dkk-3 DNA, a cytomegalovirus (CMV) promoter ligated to a site upstream of REIC/Dkk-3 DNA, and a poly A addition sequence (polyadenylation sequence, poly A) ligated to a site downstream of REIC/Dkk-3 DNA. In addition, a sequence comprising a telomerase reverse transcriptase (hTERT) enhancer, an SV40 enhancer, and a cytomegalovirus (CMV) enhancer ligated in that order (3 × enh) is ligated to a site downstream of the poly A addition sequence. Specifically, the DNA construct comprises (i) CMV promoter, (ii) REIC/Dkk-3 DNA, (iii) poly A addition sequence, and (iv) an enhancer comprising the telomerase reverse transcriptase (hTERT) enhancer, the SV40 enhancer, and the CMV enhancer ligated in that order from the 5' terminus.

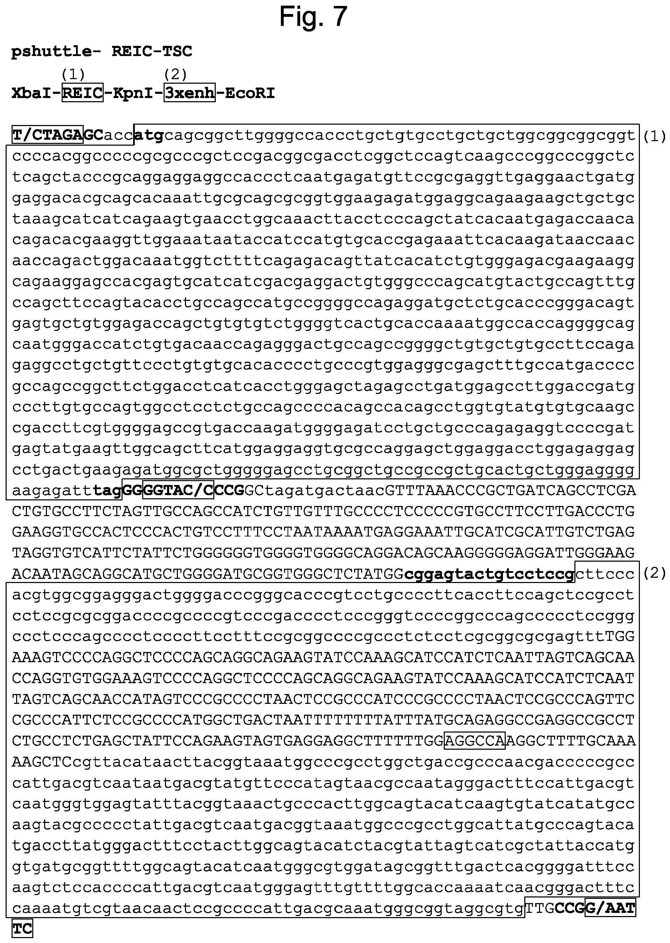

Fig. 7 and SEQ ID NO: 6 each show a partial sequence excluding the CMV promoter of the DNA construct including REIC/Dkk-3 DNA according to the present invention. In Fig. 7, the BGA poly A sequence is inserted into a site between REIC/Dkk-3 DNA and 3 × enh. The DNA construct comprising REIC/Dkk-3 DNA according to the present invention has a CMV promoter in a region upstream (on the 5' side) of the sequence represented by SEQ ID NO: 6. SEQ ID NO: 7 shows a nucleotide sequence of a region including BGH poly A and 3 enhancers contained in the above construct. In Fig. 7, regions surrounded by the frames (1) and (2) in the nucleotide sequence each indicate DNA encoding the REIC/Dkk-3 protein and 3 enhancers (3 × enh).

It is necessary that the elements described above be operably linked to each other. When such elements are operably linked to each other, each of such elements exerts its functions to increase the expression level of the target gene.

The expression cassette can be obtained by, for example, inserting REIC/Dkk-3 DNA into a pShuttle vector (Clonetech) comprising a foreign gene insertion site downstream of a commercially available CMV promoter and the BGA poly A sequence downstream of the foreign gene insertion site and ligating the telomerase reverse transcriptase (hTERT) enhancer, the SV40 enhancer, and the CMV enhancer in that order to a site downstream of the BGA poly A sequence.

The DNA construct comprising REIC/Dkk-3 DNA is as described below.
[1] A DNA construct to express the REIC/Dkk-3 protein comprising, from the 5' terminus, (i) to (iv) ligated in that order:
   (i) a CMV promoter;
   (ii) REIC/Dkk-3 DNA (a) or (b):
      (a) DNA comprising the nucleotide sequence shown in SEQ ID NO: 1; or
      (b) DNA having sequence identity of at least 90%, 95%, 97%, or 98% to the nucleotide sequence shown in by SEQ ID NO: 1;
   (iii) a poly A addition sequence; and
   (iv) an enhancer comprising the telomerase reverse transcriptase (hTERT) enhancer, the SV40 enhancer, and the CMV enhancer ligated in that order.
[2] The DNA construct according to [1], wherein the poly A addition sequence is derived from the bovine growth hormone gene (BGA poly A).
[3] The DNA construct according to [1] or [2], which comprises the nucleotide sequence shown in SEQ ID NO: 6 comprising (ii) REIC/Dkk-3 DNA, (iii) a poly A addition sequence, and (iv) an enhancer comprising the telomerase reverse transcriptase (hTERT) enhancer, the SV40 enhancer, and the CMV enhancer ligated in that order.

The DNA construct can be prepared in accordance with, for example, WO 2011/062298, US Patent Application No. 2012-0309050, WO 2012/161352, or US Patent Application No. 2014-0147917.

In the present invention, an adenovirus vector comprising REIC/Dkk-3 DNA is referred to as "Ad-REIC" or "Ad-REIC/Dkk-3."

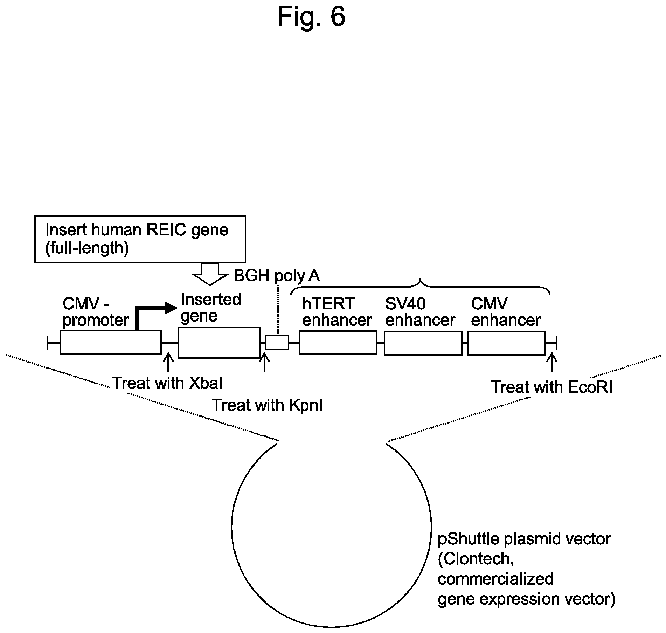

A vector system comprising the DNA construct according to the present invention is referred to as the "Super Gene Expression" (SGE) system, and an adenovirus vector comprising, for example, a DNA construct comprising REIC/Dkk-3 DNA is referred to as "Ad5-SGE-REIC/Dkk-3 (Ad-SGE-REIC)." Fig. 6 shows an example of the Ad-REIC/Dkk-3 structure and Fig. 7 shows an example of the Ad-REIC/Dkk-3 sequence.

An adenovirus vector comprising the DNA construct is obtained by introducing the DNA construct into an adenovirus vector and preparing a recombinant adenovirus. A DNA construct can be introduced into an adenovirus by introducing, for example, a DNA construct in the pShuttle vector containing the DNA construct according to the present invention into an adenovirus.

Adenovirus vectors are characterized in that: (1) gene transfection can be carried out in a variety of cells; (2) gene transfection can be efficiently performed even in cells in growth arrest; (3) they can be concentrated by centrifugation and high-titer viruses (10 to 11 PFU/ml or more) can be obtained; and (4) they are appropriate for use for direct gene transfection into *in vivo* tissue cells.

As an adenovirus vector used for gene therapy, the following vectors have been developed: a second generation adenovirus vector (Lieber, A., et al., J. Virol., 70, 8944, 1996; Mizuguchi, H. & Kay, M. A., Hum. Gene Ther., 10, 2013, 1999) obtained by deleting the E2 or E4 domain in addition to the E1/E3 domain from a first generation adenovirus vector lacking the E1/E3 domain (Miyake, S., et al., Proc. Natl. Acad. Sci. U.S.A., 93, 1320, 1996); and a third generation adenovirus vector (Steinwaerder, D. S. et al., J. Virol., 73, 9303, 1999) almost completely lacking the adenovirus genome (GUTLESS). For transfection of the gene of the present invention, any adenovirus vector may be used without particular limitation.

A recombinant adenovirus vector comprising the DNA construct containing REIC/Dkk-3 DNA according to the present invention (hereafter, it may be simply referred to as "the adenovirus vector of the present invention") may be administered to a subject, which is a human or other mammalian animal. Thus, a gene for cancer treatment is delivered to cancer cells in the subject, the gene is expressed in cancer cells, tumor cell growth is inhibited, and effects of cancer treatment are thus exerted.

The adenovirus vector of the present invention can be administered by a method that can be employed in the field of gene therapy, such as intravascular administration such as intravenous or intra-arterial administration, oral administration, intraperitoneal administration, intratracheal administration, intrabronchial administration, hypodermic administration, or percutaneous administration. In particular, the adenovirus vector of the present invention exhibits high directivity toward a particular tissue or cell and thus can efficiently deliver a target gene to a particular tissue or cell. Thus, diagnosis and treatment can be efficiently performed by intravascular administration.

The adenovirus vector of the present invention may be administered in a therapeutically effective amount. A person skilled in the field of gene therapy can readily determine a therapeutically effective amount. A dose can be adequately modified in accordance with pathological severity, sexuality, age, body weight, habit, or other factors of the subject. For example, the adenovirus vector of the present invention may be administered locally into the tumor in an amount of 0.3 × 10¹⁰ to 3 × 10¹² vp (the number of viral particles).

Use of the REIC/Dkk-3 gene or REIC/Dkk-3 DNA in the present invention encompasses the use of the REIC/Dkk-3 DNA adenovirus vector comprising REIC/Dkk-3 DNA (Ad-REIC).

Immune checkpoint inhibitors include the anti-PD-1 (programmed cell death 1) antibody, the anti-PD-L1 (programmed cell-death ligand 1) antibody, and the like. Immune checkpoint inhibitors are PD-1/PD-L1 immune checkpoint pathway inhibitors, such as the anti-PD-1 (programmed cell death 1) antibody and the anti-PD-L1 (programmed cell-death ligand 1) antibody. The PD-1/PD-L1 immune checkpoint pathway blocks T cell activation with the aid of immune checkpoint molecules such as PD-1/PD-L1 to downregulate the immune system. Such immune checkpoint molecules are known to promote antigen-specific T cell apoptosis (programmed cell death) in lymph nodes and reduce regulatory T cell (Treg) apoptosis.

The molecular-targeted agent targets a protein or gene on a cancer cell surface to attack a cancer cell.

In the present invention, as a molecular-targeted agent, a compound, such as Gefitinib, Erlotinib, Afatinib, Dacomitinib, or Osimertinib, having tyrosine kinase inhibitory activity on the epidermal growth factor receptor (EGFR), an antibody reacting with the vascular endothelial growth factor receptor (VEGFR), or the vascular endothelial growth factor (VEGF) is selected. In particular, the antibody reacting with the vascular endothelial growth factor receptor (VEGFR) exerts activity of damaging blood vessels as its dominant effects for anti-angiogenesis treatment at a high dose (clinical dose). In contrast, such antibody significantly functions for blood vessel normalization at a low dose. Thus, infiltration of tumor tissue with effector T cells is promoted, and effects of synergistically potentiating indirect effects (systemic effects) by Ad-REIC can be expected (Document: Yuhui Huanga et al., PNAS, October 23, 2012, vol. 109, no. 43, 17561-17566). In the present invention, a molecular-targeted agent exerting tyrosine kinase inhibitory activity on EGFR is referred to as the "EGFR tyrosine kinase inhibitor." An example of the anti-VEGFR antibody is Ramucirumab (tradename: Cyramza) and an example of the anti-VEGF antibody is Bevacizumab (tradename: Avastin).

In the present invention, EGFR gene mutation-positive lung cancer is the target of treatment. EGFR gene mutation-positive lung cancer is positive for the EGFR gene mutation observed in the case of non-small cell lung cancer among various types of lung cancers. It has been reported that cancer immunotherapy is less likely to be effective for EGFR gene mutation-positive lung cancer. The EGFR gene mutation plays a role in the development of cancer by activating the EGFR gene mutation signal. In the past, treatment of EGFR gene mutation-positive lung cancer had been performed with the use of the EGFR tyrosine kinase inhibitor. Such treatment was problematic since some lung cancer cells would acquire tolerance to the EGFR tyrosine kinase inhibitor, and tolerance to treatment would be developed.

In the present invention, primary and recurrent EGFR gene mutation-positive lung cancers can be treated with the use of the REIC/Dkk-3 gene, the EGFR tyrosine kinase inhibitor, the immune checkpoint inhibitor, and the molecular-targeted agent in adequate combination. The term "primary EGFR gene mutation-positive lung cancer" used herein refers to a cancer diagnosed as the "EGFR gene mutation-positive lung cancer" for the first time. The term "recurrent EGFR gene mutation-positive lung cancer" used herein refers to a cancer developed again after the combinatorial treatment of the primary cancer. More specifically, the term "primary EGFR gene mutation-positive lung cancer" refers to the EGFR gene mutation-positive lung cancer without a history of treatment with the EGFR tyrosine kinase inhibitor, and the term "recurrent EGFR gene mutation-positive lung cancer" refers to the EGFR gene mutation-positive lung cancer that has acquired tolerance to the EGFR tyrosine kinase inhibitor after the treatment with the EGFR tyrosine kinase inhibitor.

In the present invention, the primary EGFR gene mutation-positive lung cancer is treated with the use of the REIC/Dkk-3 gene in combination with the EGFR tyrosine kinase inhibitor. With the use of REIC/Dkk-3 in combination with the EGFR tyrosine kinase inhibitor, a tumor size can be further reduced, cancer antigen-specific cytotoxic CD8-positive T cells (CTLs) can be more efficiently induced, and satisfactory effects of treatment; i.e., effects of recurrence inhibition, can be expected, compared with the case of treatment performed with the use of the EGFR tyrosine kinase inhibitor by itself.

When satisfactory effects of treatment of the primary EGFR gene mutation-positive lung cancer are not attained or the cancer recurs after the treatment using the REIC/Dkk-3 gene in combination with the EGFR tyrosine kinase inhibitor, a further treatment may be performed using the REIC/Dkk-3 gene in combination with the immune checkpoint inhibitor after the treatment using the REIC/Dkk-3 gene in combination with the EGFR tyrosine kinase inhibitor. Alternatively, treatment may be performed with the use of the REIC/Dkk-3 gene, the immune checkpoint inhibitor, and the molecular-targeted agent in combination. When 3 therapeutic agents are used in combination, effects of treatment can be more significant than the effects attained by the treatment performed with the use of 2 therapeutic agents in combination.

The recurrent EGFR gene mutation-positive lung cancer may be treated with the use of 2 agents in combination; i.e., the REIC/Dkk-3 gene and the immune checkpoint inhibitor, or the use of 3 agents in combination; i.e., the REIC/Dkk-3 gene, the immune checkpoint inhibitor, and the molecular-targeted agent. When 3 therapeutic agents are used in combination, effects of treatment can be more significant than the effects attained by the treatment performed with the use of 2 agents in combination.

With the use of the REIC/Dkk-3 gene in combination with the EGFR tyrosine kinase inhibitor, the immune checkpoint inhibitor, and the molecular-targeted agent, as described above, effects of cancer treatment can be improved to a significant extent. In addition, the amount of therapeutic agents to be used can be reduced to a significant extent. As a result, side effects of anti-tumor agents can be alleviated.

When chemotherapeutic agents are used, cancer cells may exhibit resistance (tolerance) to chemotherapeutic agents. When chemotherapeutic agents are used in combination with the REIC/Dkk-3 gene, cancer cells can be effectively suppressed from developing tolerance to chemotherapeutic agents.

The immune checkpoint inhibitor and the molecular-targeted agent can be administered by a conventional technique in accordance with the instructions attached to the relevant agents. For example, the doses vary depending on symptoms, age, body weight, and other conditions. A dose of 0.001 mg to 2,000 mg, preferably 1 mg to 1,500 mg, and more preferably 5 mg to 1,000 mg may be administered in a single instance or several separate instances a day at intervals of several days, several weeks, or several months.

When the REIC/Dkk-3 gene is used in combination with the EGFR tyrosine kinase inhibitor, administration of the REIC/Dkk-3 gene can be performed simultaneously, separately, or continuously with administration of the EGFR tyrosine kinase inhibitor. Administration of the REIC/Dkk-3 gene can be performed before or after administration of the EGFR tyrosine kinase inhibitor. The REIC/Dkk-3 gene is preferably administered simultaneously with the EGFR tyrosine kinase inhibitor. In the case of simultaneous administration, they can be administered in the form of separate preparations or in the form of an anti-tumor composition containing both the REIC/Dkk-3 gene and the EGFR tyrosine kinase inhibitor.

When the immune checkpoint inhibitor and/or the molecular-targeted agent are used in combination, administration of the REIC/Dkk-3 gene can be performed simultaneously, separately, or continuously with administration of the immune checkpoint inhibitor and/or the molecular-targeted agent. Administration of the REIC/Dkk-3 gene can be performed before or after administration of the immune checkpoint inhibitor and/or the molecular-targeted agent. The REIC/Dkk-3 gene is preferably administered simultaneously with the immune checkpoint inhibitor and/or the molecular-targeted agent. In the case of simultaneous administration, they can be administered in the form of separate preparations or in the form of an anti-tumor composition containing both the REIC/Dkk-3 gene and the immune checkpoint inhibitor and/or the molecular-targeted agent. While the antibody reacting with the vascular endothelial growth factor receptor (VEGFR) can be used for the anti-angiogenesis treatment at a high dose (clinical dose), in addition, such antibody can be used in combination with the REIC/Dkk-3 gene for a therapeutic method of blood vessel normalization at a low dose. In the case of the latter, it has been reported that infiltration of tumor tissue with effector T cells can be promoted (Document: Yuhui Huanga et al., PNAS, October 23, 2012, vol. 109, no. 43, 17561-17566). Thus, effects of potentiating indirect effects (systemic effects) resulting from promotion of tumor infiltration with cytotoxic T lymphocytes (CTLs), which are effector T cells induced by the REIC/Dkk-3 gene, can be expected (Experiment 4). Specifically, Ramucirumab (tradename: Cyramza) can be administered at a dose lower than the clinically recommended dose as the anti-VEGFR antibody.

A route of administration of a composition comprising the REIC/Dkk-3 gene in combination with the EGFR tyrosine kinase inhibitor, the REIC/Dkk-3 gene in combination with the immune checkpoint inhibitor or the molecular-targeted agent, or the REIC/Dkk-3 gene, the immune checkpoint inhibitor, and/or the molecular-targeted agent in combination is not limited. Administration can be made via hypodermic, intramuscular, or intravenous injection. The EGFR tyrosine kinase inhibitor is administered orally. A systemic or local administration agent may be used. Preferably, the REIC/Dkk-3 gene is a local administration agent targeting a cancer site, and the immune checkpoint inhibitor or the molecular-targeted agent to be used in combination is a systemic or local administration agent.

A composition comprising the REIC/Dkk-3 gene in combination with the EGFR tyrosine kinase inhibitor, the REIC/Dkk-3 gene in combination with the immune checkpoint inhibitor or the molecular-targeted agent, or the REIC/Dkk-3 gene, the immune checkpoint inhibitor, and/or the molecular-targeted agent in combination contains a carrier, a diluent, and an excipient, which are usually used in the pharmaceutical field. Examples of a carrier and an excipient used for tablets include monosaccharides, such as glucose, galactose, mannose, and fructose (including sugar alcohols, such as erythritol, xylitol, sorbitol, and mannitol), disaccharides, such as lactose, maltose, sucrose, and trehalose (including sugar alcohols, such as lactitol and maltitol), oligosaccharides, polysaccharides (including thickening polysaccharides, starch, powdered cellulose, microcrystalline cellulose, dextrin, dextran, maltodextrin, and isomaltodextrin), calcium carbonate, kaolin, dibasic calcium phosphate, tribasic calcium phosphate, calcium sulfate, and magnesium stearate. Examples of aqueous liquid used for injection include physiological saline and isotonic solutions containing the saccharide such as glucose or other adjuvants, which may be used in combination with adequate solubilizers, including alcohol, polyalcohol such as propylene glycol, and nonionic surfactants. Examples of oily liquid that can be used in combination include sesame oil and soybean oil. Examples of a solubilizer that may be used in combination include benzyl benzoate and benzyl alcohol. The carrier, the diluent, and the excipient can be used independently of each other or in adequate combinations of two or more.

The REIC/Dkk-3 protein encoded by the REIC/Dkk-3 gene upregulates the anticancer immune system, so that cancer can be treated or prevented. In addition, cancer cell apoptosis is induced. Specifically, the REIC/Dkk-3 protein induces cytotoxic T lymphocytes (CTLs) and CTLs attack cancer cells throughout the body.

Cancer cells attacked by CTL express PD-L1 as the defense mechanism. The PD-1/PD-L1 immune checkpoint pathway inhibitor inhibits the defense mechanism of cancer cells. The molecular-targeted agent targets a protein or gene on a cancer cell surface to directly attack the cancer cell.

Use of the REIC/Dkk-3 gene in combination with the EGFR tyrosine kinase inhibitor and further use thereof in combination with the immune checkpoint inhibitor and/or the molecular-targeted agent exert synergistic effects in cancer treatment. Use of the REIC/Dkk-3 gene in combination with the EGFR tyrosine kinase inhibitor and use of the REIC/Dkk-3 gene in combination with the immune checkpoint inhibitor are more effective for cancer treatment than the use of each agent by itself.

The present invention includes a composition, a combinatorial agent, or a kit of combinatorial therapeutic agents comprising 2 agents in combination; i.e., the REIC/Dkk-3 gene and the EGFR tyrosine kinase inhibitor, 2 agents in combination; i.e., the REIC/Dkk-3 gene and the immune checkpoint inhibitor, or 3 agents in combination; i.e., the REIC/Dkk-3 gene, the immune checkpoint inhibitor, and the molecular-targeted agent.

The present invention also includes a method of using 2 agents in combination; i.e., the REIC/Dkk-3 gene and the EGFR tyrosine kinase inhibitor, 2 agents in combination; i.e., the REIC/Dkk-3 gene and the immune checkpoint inhibitor, or 3 agents in combination; i.e., the REIC/Dkk-3 gene, the immune checkpoint inhibitor, and the molecular-targeted agent, when preparing a therapeutic agent used for cancer treatment.

The present invention also includes a therapeutic agent comprising 2 agents in combination; i.e., the REIC/Dkk-3 gene and the EGFR tyrosine kinase inhibitor, 2 agents in combination; i.e., the REIC/Dkk-3 gene and the immune checkpoint inhibitor, or 3 agents in combination; i.e., the REIC/Dkk-3 gene, the immune checkpoint inhibitor, and the molecular-targeted agent.

The present invention further includes the REIC/Dkk-3 gene to be used in combination with the EGFR tyrosine kinase, the immune checkpoint inhibitor, or the immune checkpoint inhibitor and the molecular-targeted agent.

Further, the present invention include a method for treatment of EGFR gene mutation-positive lung cancer with the use of the therapeutic agent described above. Examples

The present invention is described in greater detail with reference to the following examples, although the present invention is not limited to these examples.

### Treatment experiment using mouse model of EGFR gene mutation-positive lung cancer

### <Materials and method>

### Construction and production of adenovirus vector

Full-length complementary DNA of the human REIC/Dkk-3 gene was integrated into a cosmid vector pAxCAwt, and the vector was then transferred into an adenovirus vector in accordance with the COS-TPC method (Takara Bio, Shiga, Japan). Subsequently, 3 enhancers; i.e., hTERT, SV40, and CMV, were added to a site downstream of the poly A sequence of the inserted REIC/Dkk-3 gene to obtain an adenovirus vector comprising the REIC/Dkk-3 gene introduced thereinto (Ad-REIC). As a control vector, an adenovirus vector comprising the LacZ gene introduced thereinto (Ad-LacZ) was used.

### Preparation of subcutaneous tumor model

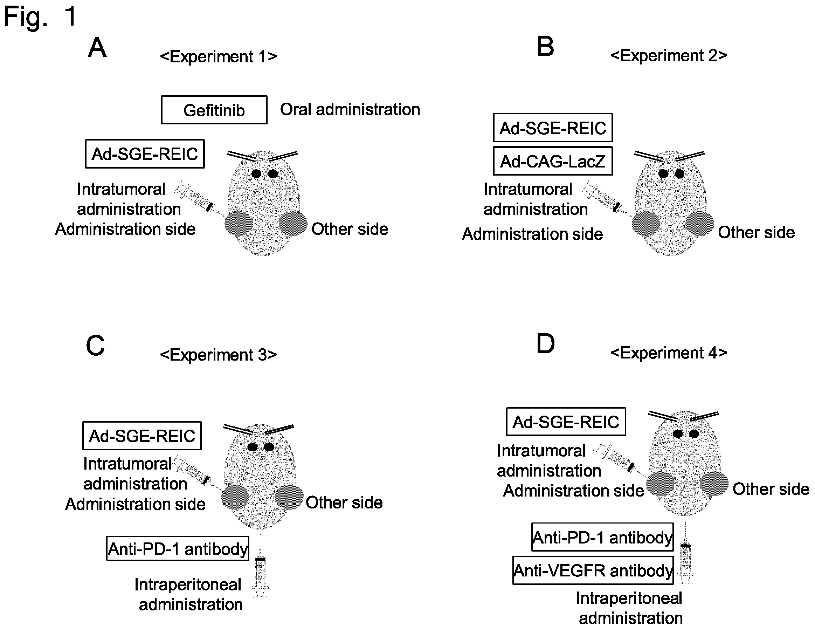

A subcutaneous tumor model was prepared by transplanting lung cancer cells of the Egfr gene mutation-positive lung cancer-carrying mouse (C57BL/6/EgfrdelE748-A752) (Ohash K. et al., Cancer Sci., 99, 1747, 2008) subcutaneously into both back regions of C57BL/6J (Higo, H. et al., Lung Cancer., 136, 86, 2019). When the tumor volume reached approximately 100 to 150 mm³, the four experiments described below were performed (Fig. 1).

### <Experiment 1>

Gefitinib (50 mg/kg/day) was administered orally for consecutive 14 days, Ad-SGE-REIC* (1.0 × 10⁹ ifu/day, days 1, 4, 7, 15, and 18) was selectively administered intratumorally on one side, and anti-tumor effects were evaluated (Fig. 1A).
* Ad-SGE-REIC: A preparation comprising the REIC/Dkk-3 gene integrated into the replication-incompetent adenovirus type 5 vector lacking the E1 region; intratumoral administration thereof leads to forced expression of REIC in cancer cells.

### <Experiment 2>

Ad-SGE-REIC (1.0 × 10⁹ ifu/day, days 1 and 8) or, as a control group, Ad-CAG-LacZ* (1.0 × 10⁹ ifu/day, day 1 and 8) was selectively administered intratumorally on one side, and anti-tumor effects were evaluated (Fig. 1B).
* Ad-CAG-LacZ: An adenovirus vector preparation not comprising the REIC/Dkk-3 gene

### <Experiment 3>

Ad-SGE-REIC (1.0 × 10⁹ ifu/day, days 1, 4, and 7) was selectively administered intratumorally on one side, and anti-tumor effects thereof attained with the use of the anti-PD-1 antibody (clone; 29F.1A12) (Bio X cell, Lebanon, NH, U.S.A.) (10 mg/kg/day, days 1 and 7, intraperitoneal administration) in combination were evaluated (Fig. 1C).

### <Experiment 4>

Ad-SGE-REIC (1.0 × 10⁹ ifu/day, days 1, 4, and 7) was selectively administered intratumorally on one side, and anti-tumor effects thereof attained with the use of the anti-PD-1 antibody (clone; 29F.1A12) (10 mg/kg/day, days 1 and 7, intraperitoneal administration) and the anti-VEGFR antibody (clone; DC101) (Bio X cell, Lebanon, NH, U.S.A.) (5 mg/kg/day^{∗}, days 1, 4, and 7, intraperitoneal administration) in combination were evaluated (Fig. 1D).
* The anti-VEGFR antibody was used at a low dose (5 mg/kg/day) as a method of blood vessel normalization treatment instead of a high dose (40 mg/kg/day) as a method of anti-angiogenesis treatment.

### Results

### <Experiment 1>

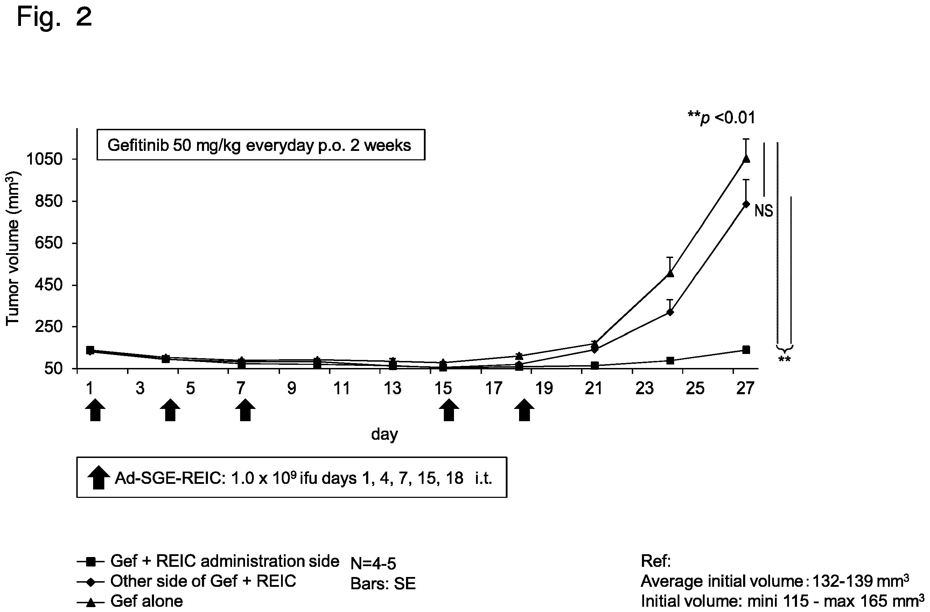

While the growth of tumors on the both sides was suppressed as a result of Gefitinib administration, tumor regrowth was observed upon termination of administration. The size of the tumor on the side to which Ad-SGE-REIC had been administered (i.e., the administration side) was apparently reduced, and regrowth was suppressed to a significant extent. On the tumor on the side opposite from the Ad-SGE-REIC administration side (i.e., the other side), in contrast, significant anti-tumor effects were not attained with the use of therapeutic agents in combination (Fig. 2).

### <Experiment 2>

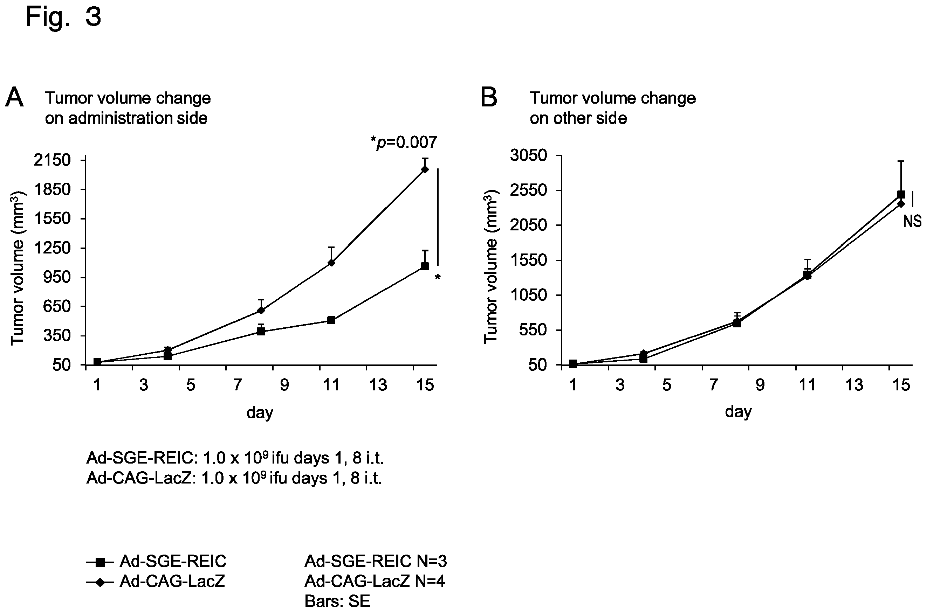

Fig. 3A shows changes in the tumor volume on the administration side and Fig. 3B shows changes in the tumor volume on the other side. Compared with the control group (Ad-CAG-LacZ), significant anti-tumor effects were observed on the tumor on the Ad-SGE-REIC administration side (Fig. 3A). On the tumor on the other side, significant anti-tumor effects were not observed (Fig. 3B).

### <Experiment 3>

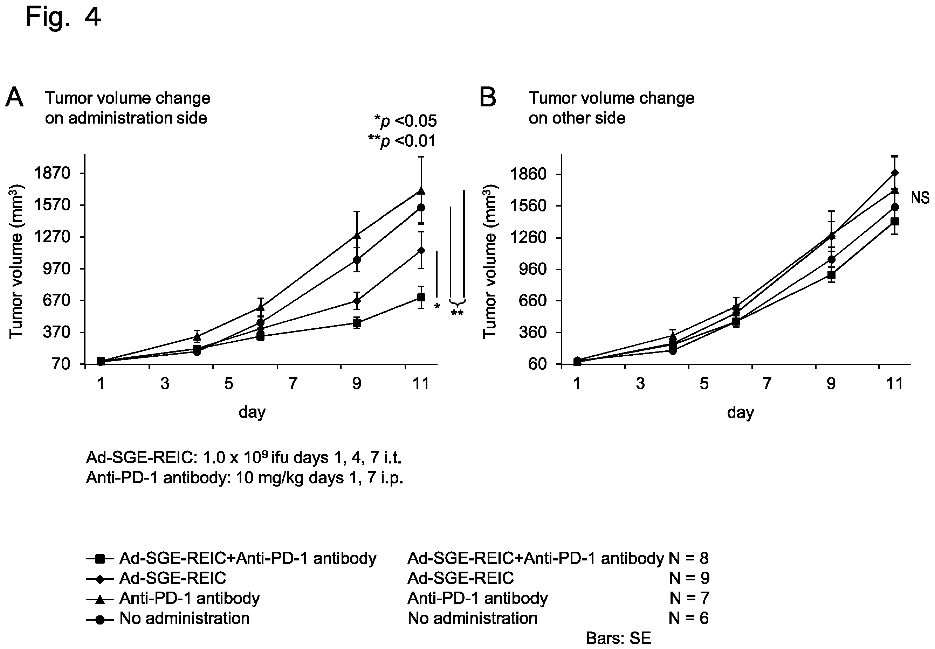

Fig. 4A shows changes in the tumor volume on the administration side and Fig. 4B shows changes in the tumor volume on the other side. While anti-tumor effects were not observed with the use of the anti-PD-1 antibody alone, more significant anti-tumor effects were exerted on the tumor on the administration side with the use of the anti-PD-1 antibody in combination with Ad-SGE-REIC, compared with the anti-tumor effects attained with the use of Ad-SGE-REIC alone (Fig. 4A). It is considered that anti-tumor effects of Ad-SGE-REIC were potentiated with the use of the anti-PD-1 antibody in combination. On the tumor on the other side, significant anti-tumor effects were not observed (Fig. 4B).

### <Experiment 4>

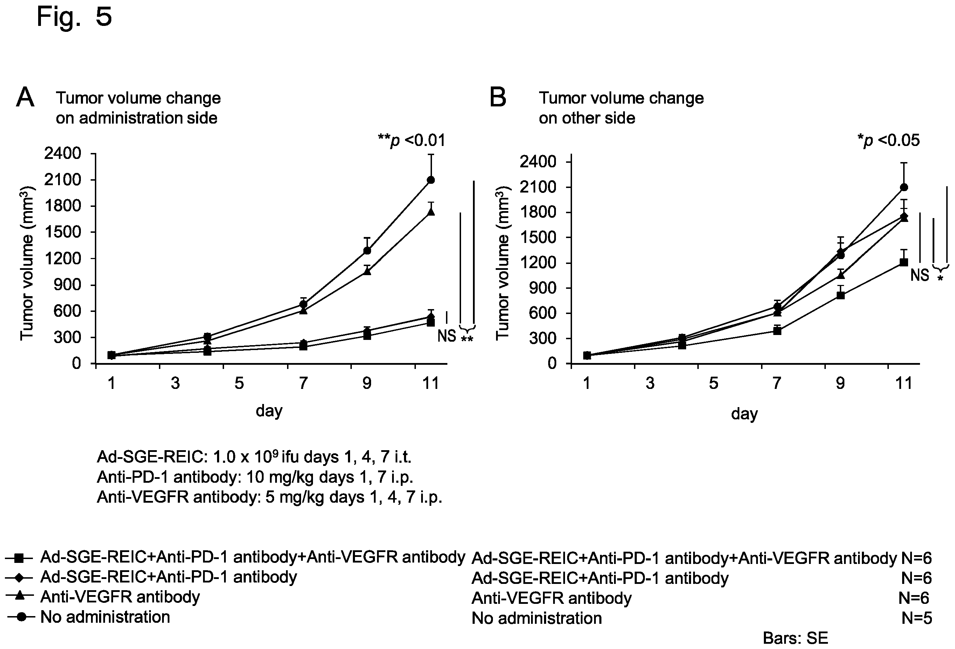

Fig. 5A shows changes in the tumor volume on the administration side and Fig. 5B shows changes in the tumor volume on the other side. With the use of Ad-SGE-REIC, the anti-PD-1 antibody, and the anti-VEGFR antibody in combination, anti-tumor effects were observed not only on the tumor on the Ad-SGE-REIC administration side (Fig. 5A) but also on the tumor on the other side (Fig. 5B).

### Industrial Applicability

With the use of the REIC/Dkk-3 gene in combination with the EGFR tyrosine kinase inhibitor or with the use of the REIC/Dkk-3 gene, the immune checkpoint inhibitor, and the molecular-targeted agent in combination, a novel therapeutic agent for treatment of EGFR gene mutation-positive lung cancer can be provided. Accordingly, use of such therapeutic agents in combination is industrially very useful. Sequence Listing Free Text

### SEQ ID NOs: 5 to 7: Synthetic sequences

All publications, patents, and patent applications cited herein are incorporated herein by reference in their entirety.

## Claims

1. A therapeutic agent comprising, as an active ingredient, the REIC/Dkk-3 gene, which is used in combination with the EGFR tyrosine kinase inhibitor for treatment of EGFR gene mutation-positive lung cancer.

2. A therapeutic agent comprising, as an active ingredient, the REIC/Dkk-3 gene, which is used in combination with the immune checkpoint inhibitor and/or the molecular-targeted agent for treatment of EGFR gene mutation-positive lung cancer.

3. The therapeutic agent according to Claim 1 or 2, wherein the target EGFR gene mutation-positive lung cancer is a primary cancer.

4. A therapeutic agent comprising, as an active ingredient, the REIC/Dkk-3 gene, which is used in combination with the immune checkpoint inhibitor for treatment of EGFR gene mutation-positive lung cancer and is to be administered to a patient of the primary EGFR gene mutation-positive lung cancer after the combinatorial treatment using the REIC/Dkk-3 gene in combination with the EGFR tyrosine kinase inhibitor.

5. A therapeutic agent comprising, as an active ingredient, the REIC/Dkk-3 gene, which is used in combination with the immune checkpoint inhibitor and the molecular-targeted agent for treatment of EGFR gene mutation-positive lung cancer and is to be administered to a patient of the primary EGFR gene mutation-positive lung cancer after the combinatorial treatment using the REIC/Dkk-3 gene in combination with the EGFR tyrosine kinase inhibitor.

6. A therapeutic agent comprising, as an active ingredient, the REIC/Dkk-3 gene, which is used in combination with the immune checkpoint inhibitor for treatment of recurrent EGFR gene mutation-positive lung cancer after the treatment using the EGFR tyrosine kinase inhibitor.

7. A therapeutic agent comprising, as an active ingredient, the REIC/Dkk-3 gene, which is used in combination with the immune checkpoint inhibitor and the molecular-targeted agent for treatment of recurrent EGFR gene mutation-positive lung cancer after the treatment using the EGFR tyrosine kinase inhibitor.

8. The therapeutic agent according to any one of Claims 2, 3, 5, and 7, wherein the molecular-targeted agent is the anti-VEGFR antibody.

9. The therapeutic agent according to any one of Claims 2 to 8, wherein the immune checkpoint inhibitor is the PD-1/PD-L1 immune checkpoint pathway inhibitor.

10. The therapeutic agent according to any one of Claims 1 to 9, wherein the REIC/Dkk-3 gene is integrated into an adenovirus vector.

11. The therapeutic agent according to any one of Claims 1 to 10, which is in the form of a local administration agent.

12. A kit of combinatorial therapeutic agents comprising the REIC/Dkk-3 gene in combination with the EGFR tyrosine kinase inhibitor, which is used for treatment of EGFR gene mutation-positive lung cancer.

13. A kit of combinatorial therapeutic agents comprising the REIC/Dkk-3 gene, the immune checkpoint inhibitor, and/or the molecular-targeted agent in combination, which is used for treatment of EGFR gene mutation-positive lung cancer.

14. The kit of combinatorial therapeutic agents according to Claim 12 or 13, wherein the target EGFR gene mutation-positive lung cancer is a primary cancer.

15. A kit of combinatorial therapeutic agents comprising the REIC/Dkk-3 gene in combination with the immune checkpoint inhibitor, which is to be administered to a patient of the primary EGFR gene mutation-positive lung cancer after the combinatorial treatment using the REIC/Dkk-3 gene in combination with the EGFR tyrosine kinase inhibitor.

16. A kit of combinatorial therapeutic agents comprising the REIC/Dkk-3 gene, the immune checkpoint inhibitor, and the molecular-targeted agent in combination, which is to be administered to a patient of the primary EGFR gene mutation-positive lung cancer after the combinatorial treatment using the REIC/Dkk-3 gene in combination with the EGFR tyrosine kinase inhibitor.

17. A kit of combinatorial therapeutic agents comprising the REIC/Dkk-3 gene in combination with the immune checkpoint inhibitor, which is used for treatment of recurrent EGFR gene mutation-positive lung cancer after the treatment using the EGFR tyrosine kinase inhibitor.

18. A kit of combinatorial therapeutic agents comprising the REIC/Dkk-3 gene, the immune checkpoint inhibitor, and the molecular-targeted agent in combination, which is used for treatment of recurrent EGFR gene mutation-positive lung cancer after the treatment using the EGFR tyrosine kinase inhibitor.

19. The kit of combinatorial therapeutic agents according to any one of Claims 13, 14, 16, and 18, wherein the molecular-targeted agent is the anti-VEGFR antibody.

20. The kit of combinatorial therapeutic agents according to any one of Claims 13 to 19, wherein the immune checkpoint inhibitor is the PD-1/PD-L1 immune checkpoint pathway inhibitor.

21. The kit of combinatorial therapeutic agents according to any one of Claims 12 to 20, wherein the REIC/Dkk-3 gene is integrated into an adenovirus vector.

22. The kit of combinatorial therapeutic agents according to any one of Claims 12 to 21, which is in the form of a local administration agent.
